# EUROPEAN PATENT APPLICATION

(11) **EP 2 716 210 A1**
(43) Date of publication of application: **09.04.2014**
(21) Application number: 13187138.6
(22) Date of filing: 02.10.2013
(51) Int. Cl.: A61B 5/055, A61B 5/06, G01R 33/28

(54) **MRI catheter with resonant filter**

(30) Priority: 03.10.2012 US 201213633931
(71) Applicant: Biosense Webster (Israel), Ltd., Yokneam 20692 (IL)
(72) Inventor: Govari, Assaf, 34400 Haifa (IL); Beeckler, Christopher Thomas, Brea, CA California 92821 (US); Papaioannou, Athanassios, Los Angeles, CA California 90066 (US)
(74) Representative: Small, Gary James

(57) **Abstract**

A medical probe (24) includes a flexible insertion tube having a distal end (34) for insertion into a body cavity and having a proximal end. The probe further includes an electrode (35) attached to the distal end of the insertion tube and configured to make electrical contact with tissue in the body cavity. An electrical lead (72) runs through the insertion tube between the distal and proximal ends. In addition a coil (74) is electrically coupled between the electrode and the lead in the insertion tube so as to define a resonant circuit having a resonant frequency in a range between 1 MHz and 300 MHz.

## Description

### FIELD OF THE INVENTION

The present invention relates generally to magnetic resonance imaging, and specifically to reducing artifacts produced during the imaging.

### BACKGROUND OF THE INVENTION

Magnetic resonance imaging (MRI) is an extremely powerful technique for visualizing tissue, particularly soft tissue, of a patient. The technique relies on exciting nuclei, typically hydrogen nuclei, from their equilibrium state, and measuring the resonant radio-frequency signals emitted by the nuclei as they relax back to equilibrium. While present-day MRI systems may provide good images, some images may include artifacts which can detract from the overall quality of the images.

Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

### SUMMARY OF THE INVENTION

An embodiment of the present invention provides a medical probe, including:
a flexible insertion tube having a distal end for insertion into a body cavity and having a proximal end;
an electrode attached to the distal end of the insertion tube and configured to make electrical contact with tissue in the body cavity;
an electrical lead running through the insertion tube between the distal and proximal ends; and
a coil electrically coupled between the electrode and the lead in the insertion tube so as to define a resonant circuit having a resonant frequency in a range between 1 MHz and 300 MHz.

Typically, the distal end is configured to function in a magnetic resonant imaging scanner operating at the resonant frequency.

In a disclosed embodiment the coil is located in the distal end.

In a further disclosed embodiment, the probe includes an irrigation tube coupled to the electrode, and the coil surrounds and is in contact with the tube. Typically, the irrigation tube is configured to convey irrigation fluid therethrough, so as to cool the coil.

In a yet further disclosed embodiment the coil is located in the distal end and is configured to generate a signal in response to a magnetic field present at the coil, and the signal is representative of a position of the distal end. The probe typically includes a processor configured to calculate the position of the distal end in response to the signal.

In an alternative embodiment the resonant frequency is between 10 MHz and 100 MHz.

Typically, the resonant frequency is selected in response to a Larmor precession frequency of nuclei of the body cavity.

In a further alternative the coil has an inductance and a self-capacitance selected in response to the resonant frequency.

In a yet further alternative embodiment the probe includes an external capacitor connected in parallel with the coil, the external capacitor having a capacitance selected in response to the resonant frequency.

There is further provided, according to an embodiment of the present invention, a method, including:
inserting a distal end of a flexible insertion tube into a body cavity, the flexible insertion tube having a proximal end;
attaching an electrode to the distal end of the insertion tube;
configuring the electrode to make electrical contact with tissue in the body cavity;
running an electrical lead through the insertion tube between the distal and proximal ends; and
electrically coupling a coil between the electrode and the lead in the insertion tube so as to define a resonant circuit having a resonant frequency in a range between 1 MHz and 300 MHz.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a system for enhanced magnetic resonance imaging (MRI), according to an embodiment of the present invention; and
Fig. 2 is a schematic cross-section of a distal end of a probe of the system, according to an embodiment of the present invention.

### DETAILED DESCRIPTION OF EMBODIMENTS

### OVERVIEW

An embodiment of the present invention provides a medical probe which is suitable for operating in a magnetic resonance imaging (MRI) environment. The probe comprises a flexible insertion tube which has a distal end for insertion into a body cavity, such as a section of a heart, and the cavity is imaged using MRI techniques. An electrode is attached to the distal end of the insertion tube so as to make electrical contact with tissue in the body cavity. The electrode may typically be used for transferring radio-frequency (RF) ablation energy to the tissue, and/or for sensing electrophysiological signals generated at the tissue.

A coil is electrically coupled between the electrode and an electrical lead which runs through the insertion tube between the distal and proximal ends of the tube. An inductance of the coil is selected so that the lead, the coil and the electrode form a resonant circuit having a resonant frequency in a range between 1 MHz and 300 MHz, typically within a range between 10 MHz and 100 MHz. Typically, the resonant frequency is selected so that it corresponds to a frequency used to generate the magnetic resonance images, i.e., to a Larmor precession frequency of nuclei in the body cavity being imaged.

Typically the electrode is perforated, so that irrigation fluid may be directed through the electrode, so as to cool the electrode and tissue in proximity to the electrode. The irrigation fluid is supplied to the electrode by a tube, and the coil may be arranged to surround and contact the tube. Typically such an arrangement is implemented by winding the coil around the tube. Arranging the tube to penetrate the coil allows the irrigation fluid to be used to cool the tube.

In some embodiments, the coil may also be used as a position sensor. The position, i.e., the location and orientation, of the distal end may be derived from signals generated if the coil is in an alternating magnetic field having a known spatial distribution.

By implementing the resonant circuit described above, the inventor has found that artifacts created in the magnetic resonance image, due to the presence of the probe distal end in the body cavity, are substantially reduced.

### DETAILED DESCRIPTION

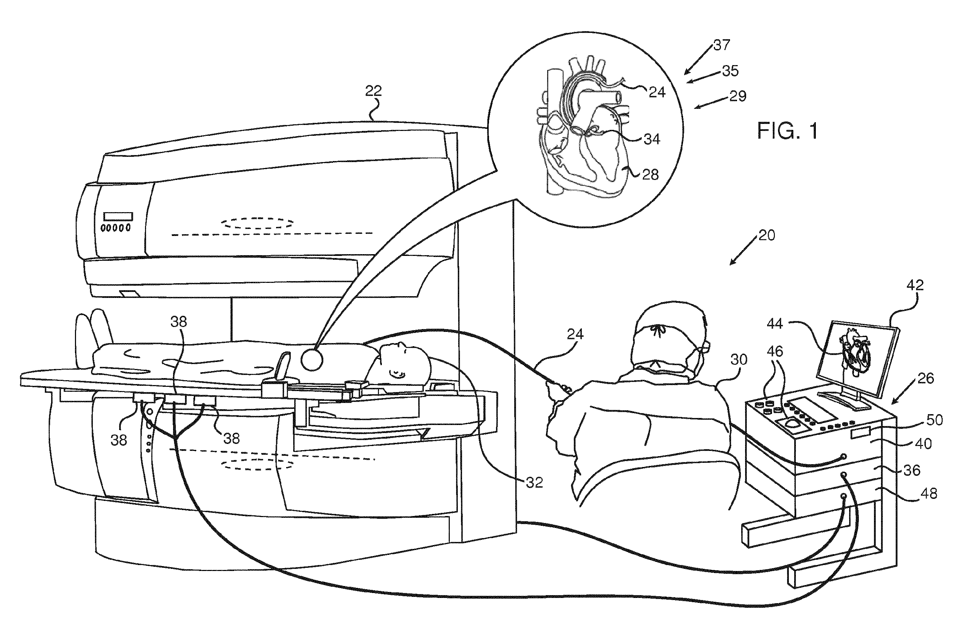

Reference is now made to Fig. 1, which is a schematic, pictorial illustration of a system 20 for enhanced magnetic resonance imaging (MRI), according to an embodiment of the present invention. System 20 comprises an MRI scanner 22, a probe 24, such as a catheter, and a control console 26. Probe 24 is configured to operate during magnetic resonance imaging of tissue in a body cavity 29 of a patient 32. By way of example the tissue of body cavity 29 that is imaged is assumed to comprise tissue of a chamber of a heart 28 of patient 32. During an MRI procedure, probe 24 is typically used for performing cardiac ablation on the tissue of heart 28, using an electrode 35 in a distal end 34 of the probe. In some embodiments, electrode 35 may be used for alternative or additional purposes, such as for mapping electrical potentials in one or more chambers of heart 28. Further alternatively or additionally, probe 24 may be used, *mutatis mutandis,* for other therapeutic and/or diagnostic functions in the heart or in other body organs.

An operator 30, such as a cardiologist, inserts probe 24 through the vascular system of patient 32 so that distal end 34 of the probe enters the cardiac chamber to be imaged.

Console 26 uses magnetic position sensing to determine orientation and location coordinates of distal end 34 inside heart 28. For the sensing, console 26 operates a driver circuit 36 that drives field generators 38, which typically comprise coils placed at known positions, e.g., below the patient's torso. A magnetic field transducer 37 that acts, and is also herein referred to, as a position sensor may be installed in distal end 34. Position sensor 37 generates electrical signals in response to the magnetic fields from the coils, thereby enabling console 26 to determine the position, i.e., the orientation and location of distal end 34, within the chamber, with respect to generators 38 and patient 32. Typically, sensor 37 comprises one or more coils.

Although in the present example system 20 measures the position, i.e., the orientation and location, of distal end 34 using magnetic-based sensors, other position tracking techniques may be used (e.g., impedance-based techniques) for measuring the position coordinates. Magnetic position tracking techniques are described, for example, in U.S. Patents 5,391,199, 5,443,489, 6,788,967, 6,690,963, 5,558,091, 6,172,499 6,177,792, whose disclosures are incorporated herein by reference. Impedance-based position tracking techniques are described, for example, in U.S. Patents 5,983,126, 6,456,864 and 5,944,022, whose disclosures are incorporated herein by reference.

A processor 40 operates scanner 22 by using circuitry and coils to form required magnetic field gradients. The processor operates other circuitry to energize transmit/receive coils of the scanner, at magnetic resonance frequencies based on the Larmor precession frequency of nuclei of cavity 29, i.e., in the example described here, of heart 28. In one embodiment the magnetic resonant frequency is approximately 63 MHz, although in other embodiments the frequency may be in a range from 1 MHz to 300 MHz, or in a narrower range between 10 MHz and 100 MHz. As is known in the art, the magnetic resonant frequency used by scanner 22 is dependent on the magnetic field generated by the magnetic field gradient coils.

Processor 40 acquires MRI data of the patient's heart 28, or at least of the cardiac chamber to be imaged, using signals received by the transmit/receive coils. The MRI data is typically collected at multiple phases of the cardiac cycle of heart 28, often (although not necessarily) over at least one cardiac cycle. Using the data, processor 40 displays an image 44 of heart 28 to operator 30 on a display 42. In some embodiments, operator 30 can manipulate image 44 using one or more input devices 46.

Processor 40 typically comprises a general-purpose computer, which is programmed in software to carry out the functions that are described herein. The software may be downloaded to processor 40 in electronic form, over a network, for example, or it may be provided on non-transitory tangible media, such as optical, magnetic or electronic memory media. Alternatively, some or all of the functions of processor 40 may be carried out by dedicated or programmable digital hardware components, or by using a combination of hardware and software elements.

Console 26 typically comprises an ablation module 48 and an irrigation module 50. The functions of these modules are explained in more detail below.

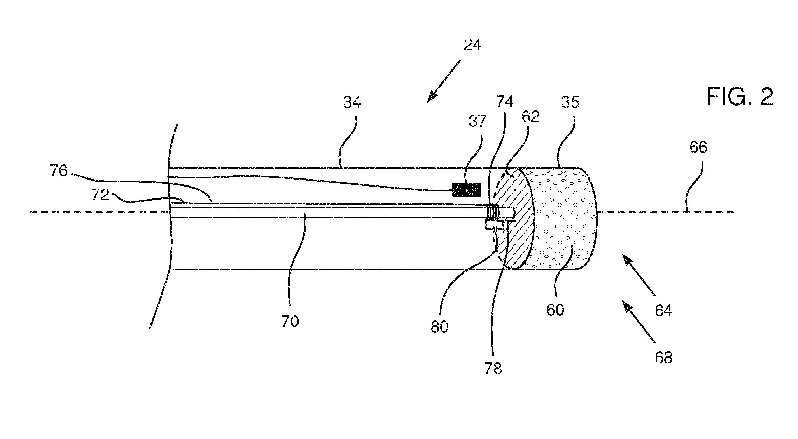

Distal end 34 is illustrated and explained with respect to Fig. 2.

Fig. 2 is a schematic cross-section of distal end 34, according to an embodiment of the present invention. Electrode 35 is attached at the distal tip of distal end 34 to probe 24. The electrode is formed as a conductive cylinder 60 which is closed at its proximal end by a first conductive circular disc 62, and at its distal end by a second conductive circular disc 64. Cylinder 60 is coaxial with an axis of symmetry 66 of distal end 34. Cylinder 60 and disc 64 are perforated by generally circular perforations 68.

In some embodiments an irrigation tube 70 is connected to disc 62, and irrigation module 36 (Fig. 1) delivers irrigation fluid to electrode 35 through the tube. The fluid enters cylinder 60 from tube 70, and exits from the cylinder via perforations 68. Tube 70 is typically, although not necessarily, coaxial with axis of symmetry 66. The irrigation fluid typically performs multiple tasks, such as cooling electrode 35 and cooling tissue being ablated by system 20.

A conductive lead 72 connects to electrode 35. Lead 72 is used to convey ablation electrical power from ablation module 48 (in console 26) to the electrode, and also to convey signals from the electrode to the console. The connection is typically, as illustrated in Fig. 2, to disc 62. A coil 74 is connected in series with lead 72, dividing the lead into a proximal section 76 and a distal section 78 that are connected by the coil. In embodiments having tube 70, coil 74 may be formed around tube 70, so that the tube penetrates the coil and is in physical contact with the coil.

In some embodiments, sensor 37 is present, as illustrated in Fig. 2, as a separate element in distal end 24. Alternatively or additionally, coil 74 is configured to act as a position sensor, as explained below.

Absent coil 74, as well as other elements of distal tip 24 described below, the presence of the distal tip in heart 28 may cause an artifact in image 44 of the heart. Typically, the artifact may appear as an enlarged image of the distal tip in image 44, while MRI scanner 22 is operative, i.e., while processor 40 is forming the magnetic field gradients and operating the transmit/receive coils described above. The inventor believes that the artifact is caused by lead 72, together with electrode 35, acting as a receiving antenna for the magnetic resonant frequency transmitted by the transmit/receive coils, and then as a re-radiating antenna for the received frequency.

In embodiments of the present invention, coil 74 may be formed so that the coil and its self-capacitance, together with lead 72 and electrode 35, form a resonant circuit resonating at the magnetic resonant frequency of scanner 22. As stated above, in embodiments of the present invention the magnetic resonant frequency may be in a range between 1 MHz and 300 MHz. The inventor has found that by forming such a resonant circuit, i.e., by selecting an inductance and self-capacitance of the coil so that lead 72, coil 74, and electrode 35 resonate at the magnetic resonant frequency, the size of any artifact produced in image 44 is substantially reduced compared with the case when no resonant circuit is present. The inventor believes that the resonant circuit reduces the magnetic frequency energy absorbed by elements of the circuit, such as electrode 35, as well as reducing re-radiation from elements of the circuit.

The magnetic resonant frequency energy absorbed by elements of the resonant circuit causes elements of the circuit to heat up, as well as heating up distal end 34. In embodiments having irrigation tube 70, the heating may be reduced or virtually eliminated by activating irrigation module 50 to force irrigation fluid through tube 70, so as to exit from perforations 60. The efficiency of the cooling effect of the irrigation fluid on coil 74 may be enhanced by arranging that the coil surrounds and is in contact with tube 70. Such an arrangement may be implemented by winding the coil around the tube.

In some embodiments, coil 74 may also be configured to act in place of, or in addition to, sensor 37. In either of these cases, for embodiments using field generators 38, processor 40 may use the voltage produced in coil 74 (from the generator fields) to establish an orientation and a location for distal end 34, substantially as described in the magnetic position tracking technique references provided above.

Typically, the self-capacitance of coil 74 acts to provide the required capacitance for the resonant circuit so there is no need for other capacitance. However, in some embodiments an optional external capacitor 80 may be connected in parallel with the coil in order to provide a capacitance needed to tune the circuit to the desired resonant frequency.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

## Claims

1. A medical probe, comprising:
a flexible insertion tube having a distal end for insertion into a body cavity and having a proximal end;
an electrode attached to the distal end of the insertion tube and configured to make electrical contact with tissue in the body cavity;
an electrical lead running through the insertion tube between the distal and proximal ends; and
a coil electrically coupled between the electrode and the lead in the insertion tube so as to define a resonant circuit having a resonant frequency in a range between 1 MHz and 300 MHz.

2. The medical probe according to claim 1, wherein the distal end is configured to function in a magnetic resonant imaging scanner operating at the resonant frequency.

3. The medical probe according to claim 1, wherein the coil is located in the distal end.

4. The medical probe according to claim 1, and comprising an irrigation tube coupled to the electrode, and wherein the coil surrounds and is in contact with the tube.

5. The medical probe according to claim 1, wherein the coil is located in the distal end and is configured to generate a signal in response to a magnetic field present at the coil, and wherein the signal is representative of a position of the distal end.

6. The medical probe according to claim 6, and comprising a processor configured to calculate the position of the distal end in response to the signal.

7. The medical probe according to claim 1, wherein the resonant frequency is selected in response to a Larmor precession frequency of nuclei of the body cavity.

8. The medical probe according to claim 1, wherein the coil has an inductance and a self-capacitance selected in response to the resonant frequency.

9. The medical probe according to claim 1, and comprising an external capacitor connected in parallel with the coil, the external capacitor having a capacitance selected in response to the resonant frequency.

10. A method, comprising:
providing a flexible insertion tube having a distal end for insertion into a body cavity, and having a proximal end;
attaching an electrode to the distal end of the insertion tube;
configuring the electrode to make electrical contact with tissue in the body cavity;
running an electrical lead through the insertion tube between the distal and proximal ends; and
electrically coupling a coil between the electrode and the lead in the insertion tube so as to define a resonant circuit having a resonant frequency in a range between 1 MHz and 300 MHz.

11. The method according to claim 10, and comprising configuring the distal end to function in a magnetic resonant imaging scanner operating at the resonant frequency.

12. The method according to claim 10, and comprising locating the coil in the distal end.

13. The method according to claim 10, and comprising coupling an irrigation tube to the electrode, wherein the coil surrounds and is in contact with the tube.

14. The medical probe according to claim 4 or method according to claim 13, wherein the irrigation tube is configured to convey irrigation fluid therethrough, so as to cool the coil.

15. The method according to claim 10, and comprising locating the coil in the distal end and configuring the coil to generate a signal in response to a magnetic field present at the coil, wherein the signal is representative of a position of the distal end.

16. The method according to claim 15, and comprising calculating the position of the distal end in response to the signal.

17. The medical probe according to claim 1 or method according to claim 10, wherein the resonant frequency is between 10 MHz and 100 MHz.

18. The method according to claim 10, and comprising selecting the resonant frequency in response to a Larmor precession frequency of nuclei of the body cavity.

19. The method according to claim 10, and comprising selecting an inductance and a self-capacitance of the coil in response to the resonant frequency.

20. The method according to claim 10, and comprising connecting an external capacitor in parallel with the coil, and selecting a capacitance of the external capacitor in response to the resonant frequency.
